# EUROPEAN PATENT APPLICATION

(11) **EP 4 276 192 A1**
(43) Date of publication of application: **15.11.2023**
(21) Application number: 22172665.6
(22) Date of filing: 10.05.2022
(51) Int. Cl.: C12Q 1/25, C12Q 1/34, G01N 33/574, A61P 35/02

(54) **ANALYTICAL METHOD AND THERAPEUTICAL AGENT FOR USE IN COMBINATION WITH L-ASPARAGINASE IN TUMOUR THERAPY**

(71) Applicant: Medizinische Hochschule Hannover, 30625 Hannover (DE)
(72) Inventor: Hinze, Laura, 30625 Hannover (DE); Ibrahim, Nurul Khalida, 30625 Hannover (DE)
(74) Representative: Taruttis, Stefan Georg

(57) **Abstract**

The invention provides an analytical method for detecting the level of activity of superoxide dismutase in a sample originating from a patient for determining the sensitivity for, or resistance against, tumour treatment with L-asparaginase. Further, the invention provides an inhibitor of SOD2 and/or an inhibitor of UBR2 and/or of UBR1 in combination with L-asparaginase for use in the treatment of a tumour, optionally in combination with a chemotherapeutic agent.

## Description

The present invention provides an analytical method for use in combination with tumour treatment making use of L-asparaginase as a therapeutical agent, and provides a therapeutical agent for use in combination with the use of L-asparaginase in tumour therapy.

The analytical method of the invention is suitable for predicting the outcome of tumour therapy by administration of L-asparaginase, e.g. for stratifying patients into a group that is predicted to be susceptible to treatment with L-asparaginase, and another group that is predicted as having a poorer response to treatment with L-asparaginase. This stratification of patients allows the therapeutical agent of the invention to be used in combination with L-asparaginase in the treatment of tumour. The preferred tumour to be analysed and for use of the therapeutic agent in combination with L-asparaginase is one of colorectal cancer and leukemia and, especially acute lymphoblastic leukemia (ALL). Generally herein, tumour and cancer are used interchangeably.

### State of the art

Appel et al., Blood 107, 4244-4249 (2006) analysed the effect of administration of L-asparaginase and PEGylated L-asparaginase in treatment of ALL on the expression of asparagin synthetase. The therapeutic effect of administration of L-asparaginase against cancer cells is seen in the depletion of serum asparagine by enzymatic conversion to aspartate.
Place et al., Lancet Oncol, 1677-1690 (2015) analysed the effects of treatment of ALL with native L-asparaginase and PEGylated L-asparaginase.
Hinze et al., Cancer Discov. 10(11) 1690-1705 (2021) describe the analysis of the WNT/β-catenin signaling pathway in colorectal cancer cell lines which originally were resistant to treatment with L-asparaginase. It was found that treatment of these cells by inhibition of GSK3α induced activation of WNT-signaling, which lead to sensitization of the cancer cells to treatment with L-asparaginase.

### Object of the invention

It is an object of the invention to provide an analytical method for predicting the efficacy, or outcome, of treatment of cancer, especially of ALL, with L-asparaginase, especially to identify patients having a cancer, e.g. ALL, that can be expected to be resistant against L-asparaginase therapy. A further object is to provide an improved treatment of cancer using administration of L-asparaginase, especially for treatment of cancer that has been analysed and predicted to have resistance against L-asparaginase treatment.

### Description of the invention

The invention achieves the object by the features of the claims, especially by an analytical method for detecting the level of activity of superoxide dismutase (SOD2, e.g. UniProtKB P04179 (SODM_HUMAN) in a sample originating from a patient for determining the sensitivity for, or resistance against, tumour treatment with L-asparaginase. In this embodiment, the invention provides a method for predicting the efficacy of the use of L-asparaginase for use in tumour treatment, e.g. for determination of suitability of use of L-asparaginase in tumour treatment, and respectively for stratifying patients for tumour therapy by L-asparaginase treatment, or for tumour therapy without L-asparaginase treatment, e.g. irradiation therapy and/or chemotherapy only. The sample preferably comprises or consists of cancer cells, e.g. colorectal cancer cells or leukemia cells isolated from a blood sample originating from the patient. Therein, the level of activity of SOD2 can be e.g. the level of SOD2-K68 acetylation, which can be determined by FACS or Western blotting using immunostaining by an antibody specific for acetylated SOD2-K68, or by a colorimetric assay, e.g. use of a superoxide dismutase assay kit. A high level of activity of SOD2 indicates that the cancer cells are not sensitive, e.g. resistant, to treatment with L-asparaginase, predicting a poor effect of L-asparaginase in anti-cancer treatment. In contrast, a low activity level of SOD2 indicates that the cancer cells are sensitive against treatment with L-asparaginase, predicting effectivity of treatment of cancer cells by L-asparaginase. Accordingly, preferably only patients with a low level of activity of SOD2 in cancer cells are selected for therapy that comprises L-asparaginase for use in treatment. Patients with a high activity of SOD2 in cancer cells are selected for therapy that comprises other pharmaceuticals than L-asparaginase for use in treatment.

A low activity level of SOD2 preferably is an activity of SOD2 as found in cancer cells that are sensitive to treatment with L-asparaginase, e.g. an activity below 50%, below 40%, preferably below 35% or below 30% of the activity of SOD2 in cancer cells that are resistant to L-asparaginase.

Further, the invention provides an inhibitor of SOD2 and/or an inhibitor of UBR2 and/or of UBR1 in combination with L-asparaginase for use in the treatment of a tumour, optionally in combination with a chemotherapeutic agent.

The invention shows that an inhibitor of SOD2 and, separately an inhibitor of UBR2 increases the sensitivity of cancer cells for L-asparaginase. Accordingly, an inhibitor for SOD2 and/or an inhibitor for UBR2 and/or UBR1 in combination with L-asparaginase is suitable for use in the treatment of a tumour.

As UBR1 (E3 ubiquitin-protein ligase 1, UniProt KB: Q8IWV7 (UBR1_HUMAN)) and UBR2 (E3 ubiquitin-protein ligase 2, UniProt KB: Q8IWV8 (UBR2 HUMAN)) have 95% amino acid identity, the increased sensitivity of cancer cells in the presence of an inhibitor of UBR2 also indicates that a inhibitor of UBR1 increases sensitivity of the cancer cells for treatment by L-asparaginase.

Currently, it is assumed that sensitization of cancer cells to treatment with L-asparaginase by each of SOD2 and UBR2 and its close relative UBR1 is independent of reactive oxygen species, of cell cycle changes, of alterations of mTOR signaling, and of glutamine anaplerosis. The mechanism that causes sensitization of cancer cells to treatment with L-asparaginase by each of SOD2 and/or UBR2 and UBR1 is currently assumed to be a regulation of protein degeneration by the N-degron pathway, also referred to as N-end rule pathway. Inhibition of SOD2 and/or UBR2/UBR1 is believed to reduce activity of the protein degradation, which protein degradation may be activated in response to L-asparaginase treatment in order to release amino acids to replenish asparagine intracellularly, leading to resistance against L-asparaginase. The inhibition of SOD2 and/or of one or both of UBR2 and UBR1 in combination with administration of L-asparaginase according to the invention is assumed to hinder the release of amino acids by protein degradation in the N-degron pathway, thus supporting the starvation of cancer cells of asparagine due to L-asparaginase.

The invention is now described by way of examples with reference to the figures that show in
- Fig. 1A activity levels of SOD2 in human leukemia cells that are sensitive or resistant to treatment with L-asparaginase,
- Fig. 1B activity levels of SOD2 in human cancer cells, present as xenografts on mice, which cancer cells are sensitive or resistant to treatment with L-asparaginase,
- Fig. 1C the receiver operating characteristic (ROC) analysis of SOD2 activity as a biomarker of susceptibility of cancer cells to L-asparaginase treatment,
- Fig. 2A viability of CCRF-CEM cancer cells after knock-down of SOD2 in L-asparaginase treatment,
- Fig. 2B viability of Jurkat cancer cells after knock-down of SOD2 in L-asparaginase treatment,
- Fig. 2C viability of MOLT4 cancer cells after knock-down of SOD2 in L-asparaginase treatment,
- Fig. 2D viability of NALM-16 cancer cells after knock-down of SOD2 in L-asparaginase treatment,
- Fig. 2E viability of HCT-15 cancer cells after knock-down of SOD2 in L-asparaginase treatment,
- Fig. 2F viability of SW480 cancer cells after knock-down of SOD2 in L-asparaginase treatment,
- Fig. 3A cancer cell viability in transduced cancer cells in presence of Vincristine,
- Fig. 3B cancer cell viability in transduced cancer cells in presence of Dexamethasone,
- Fig. 3C cancer cell viability in transduced cancer cells in presence of Doxorubicin,
- Fig. 3D cancer cell viability in transduced cancer cells in presence of 6-mercaptopurine,
- Fig. 4A viability of Jurkat cancer cells after knock-down of UBR2 in L-asparaginase treatment, and
- Fig. 4B viability of CCRF-CEM cancer cells after knock-down of UBR2 in L-asparaginase treatment.

Generally, statistical significance P was assessed by a two-sided Student's t-test with Welch adjustment, or by a one-way ANOVA with Dunnett's adjustment for multiple comparisons unless otherwise indicated.

### Example 1: Activity level of SOD2 in cancer cells as indicator of sensitivity for L-asparaginase treatment and inhibition of SOD2 increases sensitivity in L-asparaginase treatment

As representatives of cancer cells, leukemia cell lines (CCRF-CEM, Jurkat, MOLT4, DND41, Loucy, KOPTK1) were used. The cell lines were cultivated and treated with 100 U/L L-asparaginase in the medium, or vehicle (PBS) as control with incubation under cell culture conditions for 48 h.

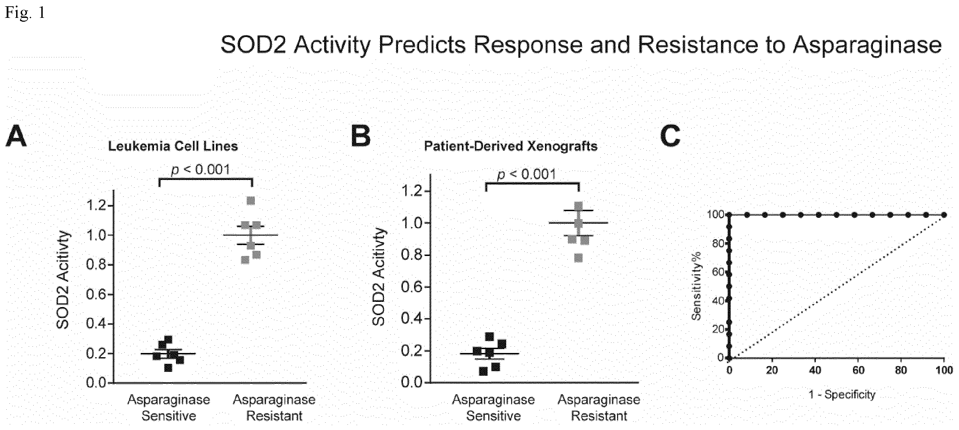

Analysis of the activity level of SOD2 was by Western blotting of total cell proteins, using immunostaining for SOD2-K68 acetylation with antibody (available from Abcam, catalog number 137037), with quantitative detection of the blot, or by a colorimetric assay, e.g. the Superoxide Dismutase Assay Kit (Cayman 706002) The results are depicted in Fig. 1A, showing that low levels of SOD2 activity, e.g. below 40% of the SOD2 activity level found in L-asparaginase resistant cells, correlates with sensitivity to L-asparaginase treatment, whereas high levels of SOD2 activity correlate with resistance against L-asparaginase treatment.

For treatment of PDX cells, ALL clinical specimens collected from children enrolled on ALL-BFM 2000, COALL 0703, COALL 06-97, or AIEOP-BFM ALL 2009 were used, with informed consent and institutional review board approval in accordance with the Declaration of Helsinki. Patient-derived xenografts were generated by engrafting viably frozen leukemic cell into immunodeficient mice followed by harvesting and viably freezing, as described in Townsend et al., 2016. PDX cells were thawed, and subsequently cultured in vitro for treatment with vehicle (PBS), or asparaginase (100 U/L) for 48 hrs to assess drug response.

The results show that low activity of SOD2 in the cancer cells correlates with sensitivity to L-asparaginase treatment, whereas a high level of activity of SOD2 correlates with resistance to L-asparaginase.

Fig. 1C for the data shown in Figures 1A and 1B depicts the receiver operating characteristic analysis of SOD2, showing that the activity of SOD2 in cancer cells is a bioindicator suitable for predicting the efficacy of treatment with L-asparaginase.

As a further proof of the correlation of low SOD2 activity in cancer cells with sensitivity for L-asparaginase treatment, resp. of the correlation of high SOD2 activity in cancer cells with resistance against L-asparaginase treatment, T-ALL cells CCRF-CEM, and Jurkat cells, as well as B-ALL cells (NALM-16) and colorectal cancer cells HCT-15, and SW480 were transduced with shRNA that knocks-down SOD2 (shSOD2), or transduced with shRNA that knocks-down luciferase as a control (shLuc), and treated with 0.1 U/L, 1 u/L, 10 U/L, 100 U/L, or 1000 U/L L-asparaginase, followed by incubation under cell culture conditions for 8 days. After the incubation, cell viability was assessed by counting viable cells by Trypan blue exclusion assay. For knock-down of SOD2, shRNA TRCN0000005942 (mature antisense sequence ATAAGGCCTGTTGTTCCTTGC, SEQ ID NO: 1 (shSOD2 #3) or TRCN0000005939 (mature antisense sequence AAAGAGCTTAACATACTCAGC, SEQ ID NO: 2) (shSOD2 #4) were used.

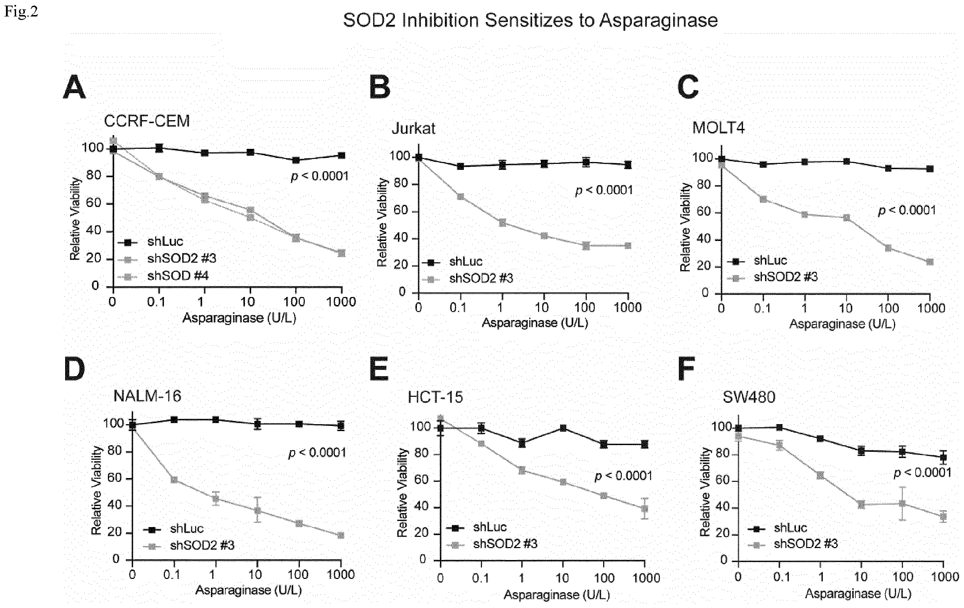

Fig. 2A-F for the different cancer cells depict the anti-cancer activity of L-asparaginase for the cells after shRNA (shSOD) mediated decrease of activity of SOD2, whereas the control-transduced (shLuc) cancer cells continued to be resistant against L-asparaginase treatment. P-values of Fig. 2A-F reflect the comparison of shLuc and shSOD2 at a dose of 100 U/L L-asparaginase and were calculated using a one-way ANOVA with Dunnet's adjustments for multiple comparisons.

These results show that in several types of cancer the sensitivity for L-asparaginase treatment correlates with low activity of SOD2, and that inhibition of activity of SOD2 in cancer increases the sensitivity of the cancer for L-asparaginase. As the results of Example 2 show that the inhibition of SOD2 specifically increases the efficacy of L-asparaginase in the treatment of a tumour, an inhibitor for SOD2 is effective in combination with L-asparaginase alone or in combination with L-asparaginase and a further chemotherapeutic agent for use in treatment of a tumour.

Further, these results show that an inhibitor for SOD2, herein represented by the shRNA that knocks-down SOD2 expression in the cancer cells, is suitable in combination with L-asparaginase for use in the treatment of cancer.

### Example 2: Effect of chemotherapeutic agents for use in treatment of cancer not affected by inhibition of SOD2

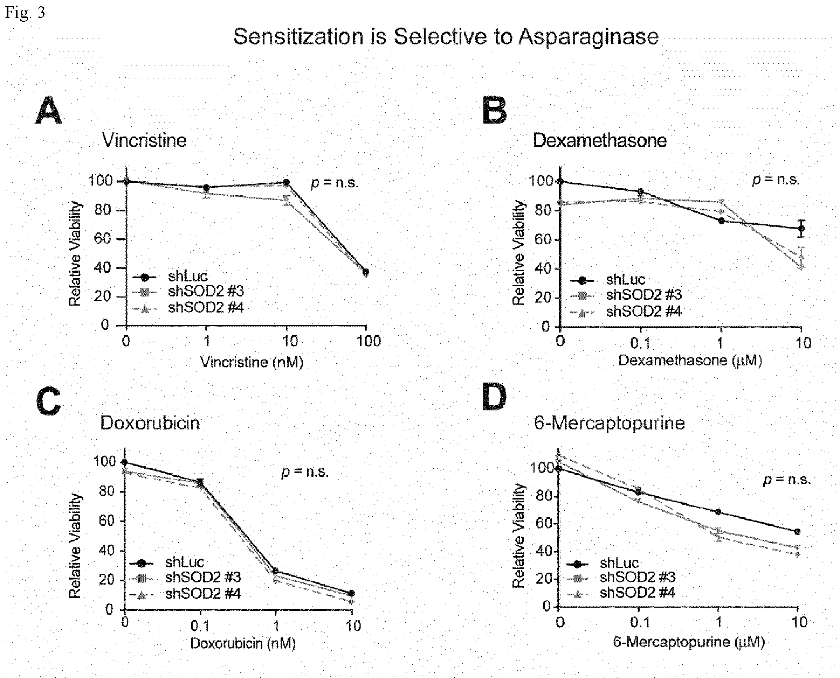

As an example for cancer cells, T-ALL cells (CCRF-CEM) were used in in vitro experiments with a chemotherapeutic agent for treatment of cancer. The cancer cells were transduced with shRNA (shSOD2 #3 or SOD2 #4) that reduces or knocks-down expression of SOD2, and shLuc as comparative control (original SOD2 activity maintained). As the chemotherapeutic agent, Vincristine, Dexamethasone, Doxorubicin, or 6-mercaptopurine were used. Following incubation under cell culture conditions for 8 days, cell viability was analysed by Trypan blue exclusion. In Fig. 3A-D, P-values reflect the comparison of cancer cells transduced by shLuc or shSOD2 at the highest dosage of the chemotherapeutic agent.

The results are depicted in Fig. 3A-D, showing that inhibition of SOD2 as caused by knock-down of SOD2 in cancer cells specifically correlates with their sensitivity for L-asparaginase, whereas sensitivity for these chemotherapeutic agents is essentially not affected by the activity level of SOD2.

### Example 3: L-asparaginase cancer in combination with an inhibitor of UBR2 for use in treatment of cancer

Cas9-expressing Jurkat cells or T-ALL cells (CCRF-CEM) were treated with sgRNA that specifically knocked-out UBR2 (sgUBR2 #3, GAAACTTGAAATAGTCTAAA, SEQ ID NO: 3) or control sgRNA directed against the safe harbor control locus AAVS1 (sgAAVS1, 5'-AGCGGCTCCAATTCGGAAGT-3', SEQ ID NO: 4), and after knock-out of UBR2 cells were incubated under cell-culture conditions with 100 U/L L-asparaginase (Asp) or PBS as control agent (Vehicle). Cell viability was analysed by Trypan blue exclusion after 48 h of incubation.

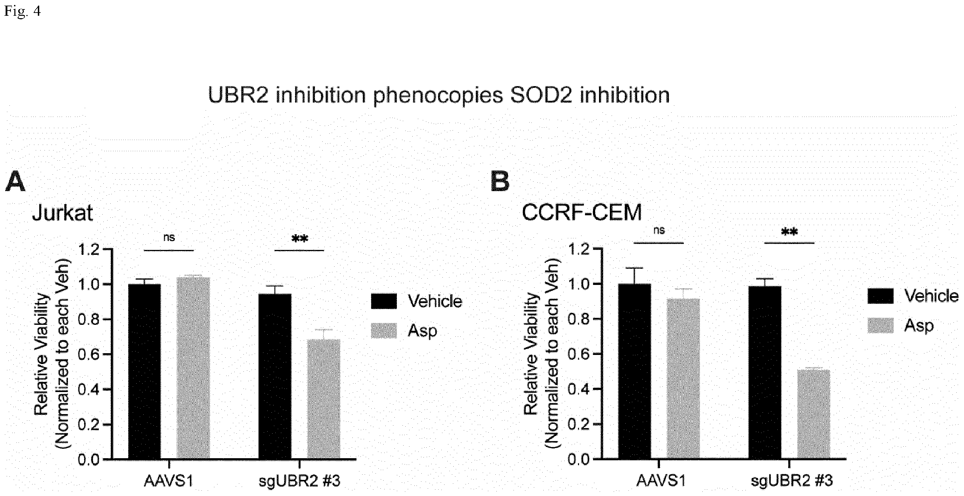

Fig. 4A for Jurkat cells, Fig. 4B for the T-ALL cells depict the cell viability normalized to the cell viability for Vehicle. The results show that the inhibitor for UBR2, represented by the sgRNA (sgUBR2 #3) for knock-down of UBR2, increased the sensitivity of these cells for treatment with L-asparaginase, whereas control inhibitor (AAVS 1) essentially had no effect on the sensitivity towards L-asparaginase. P-values reflect the comparison of control inhibitor (AAVS 1) and inhibitor of UBR2 (sgUBR2), calculated using a two-way ANOVA with Sidak's adjustments for multiple comparisons.

## Claims

1. Method for analysis of sensitivity for, or resistance against, tumour treatment with L-asparaginase in a sample originating from a patient, **characterized by** analysing the activity of superoxide dismutase 2 (SOD2).

2. Method according to claim 1, **characterized in that** the activity of SOD2 is determined as the concentration of K68 acetylation of SOD2, or a colorimetric superoxide dismutase assay

3. Method according to one of the preceding claims, wherein a high activity of SOD2 indicates resistance of the tumour against treatment with L-asparaginase, and a low activity of SOD2 indicates susceptibility of the tumour in treatment with L-asparaginase.

4. L-asparaginase for use in the treatment of a tumour, **characterized by** a combination of L-asparaginase with an inhibitor of SOD2 and/or an inhibitor of UBR2 and/or of UBR1 for use in the treatment of a tumour.

5. L-asparaginase in combination with an inhibitor of SOD2 and/or an inhibitor of UBR2 and/or of UBR1 for use in the treatment of a tumour according to claim 4, **characterized in that** the tumour was analysed to have a high activity of SOD2.

6. L-asparaginase in combination with an inhibitor of SOD2 and/or an inhibitor of UBR2 and/or of UBR1 for use in the treatment of a tumour according to one of claims 4 to 5, **characterized in that** the tumour is ALL or colorectal cancer.
